(19) Europäisches Patentamt
European Patent Office
Office européen des brevets

(11) **EP 3 786 153 B1**

(12) **EUROPEAN PATENT SPECIFICATION**

(45) Date of publication and mention of the grant of the patent:
**19.07.2023 Bulletin 2023/29**

(21) Application number: **20153984.8**

(22) Date of filing: **28.01.2020**

(51) International Patent Classification (IPC):
*C07C 303/22* (2006.01)  *C07C 303/44* (2006.01)
*C07C 309/14* (2006.01)  *B01D 15/00* (2006.01)

(52) Cooperative Patent Classification (CPC):
(C-Sets available)
**B01D 15/363; B01J 41/04; B01J 47/014;
B01J 49/57; C07C 303/22; C07C 303/44**  (Cont.)

(54) **METHOD AND SYSTEM FOR REMOVING IMPURITIES OUT OF TAURINE MOTHER LIQUOR AND TAURINE MOTHER LIQUOR RECOVERY**

VERFAHREN UND SYSTEM ZUR ENTFERNUNG VON VERUNREINIGUNGEN AUS TAURIN-MUTTERLAUGE UND RÜCKGEWINNUNG VON TAURIN-MUTTERLAUGE

PROCÉDÉ ET SYSTÈME POUR L'ÉLIMINATION DES IMPURETÉS D'UNE LIQUEUR MÈRE TAURINE ET RÉCUPÉRATION D'UNE LIQUEUR MÈRE TAURINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.09.2019 CN 201910826732**

(43) Date of publication of application:
**03.03.2021 Bulletin 2021/09**

(73) Proprietor: **Qiangjiang Yongan Pharmaceutical
Co., Ltd.**
**Qianjiang, Hubei 433100 (CN)**

(72) Inventors:
• **CHEN, Yong**
**Qianjiang, Hubei 433100 (CN)**
• **FANG, Xiquan**
**Qianjiang, Hubei 433100 (CN)**

• **LIU, Feng**
**Qianjiang, Hubei 433100 (CN)**
• **LI, Shaobo**
**Qianjiang, Hubei 433100 (CN)**

(74) Representative: **Gulde & Partner
Patent- und Rechtsanwaltskanzlei mbB
Wallstraße 58/59
10179 Berlin (DE)**

(56) References cited:
**EP-A1- 3 415 497    US-A- 2 693 488**

EP 3 786 153 B1

(52) Cooperative Patent Classification (CPC): (Cont.)

C-Sets
**C07C 303/22, C07C 309/14;**
**C07C 303/44, C07C 309/14**

**Description**

**Field of Invention**

This invention relates to a production method for chemically synthesizing taurine, in particular to a mother liquor produced during the production of taurine by the ethylene oxide method, a method for removing impurities and recycling the mother liquor, and a production system thereof.

**Background of Invention**

The chemical name of taurine, which is 2-aminoethanesulfonic acid, is the most abundant sulfur-containing free amino acid in the body's cells. The chemical synthesis process line of taurine mainly includes ethylene oxide method and ethanolamine method. Among them, the ethylene oxide process includes three steps:

(1) Using ethylene oxide as the starting material, the addition reaction of ethylene oxide and sodium bisulfite to generate sodium isethionate;

Main Reaction :

$$CH_2CH_2O + NaHSO_3 \text{ --+ } HOCH_2CH_2SO_3Na$$

$$HOCH_2CH_2SO_2Na + NH_3 \text{ -> } H_2NCH_2CH_2SO_2Na + H_2O$$

$$2H_2NCH_2CH_2SO_2Na + H_2SO_4 \text{ -> } 2H_2NCH_2CH_2SO_3H + Na_2SO_4$$

Addition Side Reaction:

$$CH_2CH_2O + H_2O \text{ -> } HOCH_2CH_2OH$$

(2) Ammonolysis of sodium isethionate to generate sodium taurine;
Ammonia hydrolysis side reactions:

$$2HOCH_2CH_2SO_3Na + NH_3 \rightarrow HN(CH_2CH_2SO_3Na)_2 + 2H_2O$$

$$3HOCH_2CH_2SO_3Na + NH_3 \rightarrow N\,(CH_2CH_2SO_3Na)_3 + 3H_2O$$

(3) Taurine is generated through acidification, such as hydrochloric acid, is preferably neutralized with sulfuric acid to generate taurine and inorganic salts.

By-products are inevitably produced in the above addition and synthesis reactions, including ethylene glycol, ethylene glycol polymers and the like. The ammonolysis reaction is a reversible reaction, about more than 20% of sodium isethionate will enter the next process with the production system, after the ammonia solution is neutralized by sulfuric acid, the mother liquor is separated and concentrated for 1-3 times, and then the last mother liquor is generated. The impurities in the last mother liquor mainly include taurine, sodium isethionate, sodium sulfate, sodium iminodisulfonate, ethylene glycol and polyethylene glycol, and trace metal ions, which are highly polluting emissions. When the existing production method adopts the mother liquor circulation, the cumulative increase of by-products will occur, when the by-product reaches a threshold, it can only be solved by discharging part of the mother liquor but resulting in waste and pollution.
Chinese patents CN101508657, CN10158658, CN10158659 and CN101486669 describe a method for neutralizing sodium taurine by sulfuric acid to generate taurine and sodium sulfate. After cooling, the crude taurine can be easily generated by filtering the crystal suspension. However, the waste mother liquor still contains taurine, sulfate and other organic impurities.
On the use of mother liquor, "Research on Taurine Ammonolysis Process", published in Issue 5, Volume 44 of Shandong Chemical Industry, 2015, (Author: Liu Fuming, Xie Limin), specifies in detail the process of taurine reaction, and the other organic impurities in the reaction, such as ethylene glycol and polyethylene glycol, and analyzes the effect of mother liquor application on yield. The higher the content of mother liquor in the reaction system, the higher the product yields. In the actual production process, the amount of mother liquor cannot be infinitely increased, as the content of mother liquor increases, by-products in the reaction system increase greatly, and the output of the last mother liquor in the production process can only meet the maximum 9.0% (v/v) set of dosage. Considering comprehensive production costs and yield quality, it is most appropriate to choose the mother liquor content of 6.3% - 8.3% (v/v). Therefore, the removal of impurities in the mother liquor is a prerequisite for increasing the application of the mother liquor, otherwise the increase in the amount of application will cause more by-products in production and the production will be more unstable.

Chinese patent CN107056659A and European patent EP 3415497 A1 describe a method for neutralizing sodium taurine by ion exchange to generate taurine, and then recycling the mother liquor to further increase the yield. This process route mainly avoids the generation of sulfate, recycles sodium atoms therein, and greatly saves raw materials such as sulfuric acid and sodium hydroxide, wherein the extracted mother liquor is returned to the ammonolysis reaction as a raw material. However, the process does not avoid the occurrence of side reactions such as addition and synthesis, and the mother liquor still needs to be subjected to removing impurity treatment.

With regard to the removing impurity treatment of the mother liquor, Chinese patent CN105732440 discloses a method for producing taurine by fully recovering the mother liquor, which mainly removes impurities by neutralization in the second stage to obtain crude taurine, and the mother liquor is subjected to pressure filtration and catalysis to further remove sulfuric acid, after that, the sodium is reused in the synthesis section. Among them, the removal effect of ethylene glycol and other organic polymers therein by the second-stage neutralization and removing impurities is limited.

In summary, although the current taurine preparation process is relatively mature, there are still many deficiencies in the separation and purification of taurine and the recycling of mother liquor, and an effective solution is urgently needed.

## Summary of Invention

The purpose of this invention is to provide a removing impurity and recovery process and a production device, which can effectively remove impurities in the last mother liquor of taurine and recycle them, so as to apply the last mother liquor to the synthesis section and reduce environmental pollution caused by discharging of mother liquor to improve product yield.

Through a lot of researches and experiments, the inventors have unexpectedly discovered a method for removing impurities and recovering taurine mother liquor, which can recover all the mother liquor in the existing ethylene oxide process for preparing sulfuric acid after removing impurities.

The technical solution of this invention to solve the said technical problems is as follows: a method for removing impurities and recovering taurine mother liquor, which is applied to the taurine production process of the ethylene oxide method, to process the last mother liquor of taurine, and the last mother liquor of taurine refers to the taurine mother liquor that has been concentrated and separated at least once. It includes the following processes:

(a) the last mother liquor of taurine is ion-exchanged through anion exchange resin, and the effective components are adsorbed on the anion exchange resin; then the anion exchange resin is eluted and regenerated with alkali solution, and the outlet liquor is collected;

(b) ammonia mixing treatment is performed on the outlet material liquor collected in step (a), and solid-liquor separation is performed to generate mother liquor after removing impurities;

(c) the mother liquor generated in step (b) enters the ammonolysis step.

Preferably, during the said ammonia mixing treatment, liquor ammonia or ammonia gas is added to the said outlet material liquor, and the mass-volume ratio of ammonia is 15g/100ml or more.

Preferably, the anion exchange resin is alkaline anion exchange resins.

Preferably, before step (a), performing decolorization and removing impurity treatment on the last mother liquor of taurine with activated carbon, comprises:

the activated carbon is added to the last mother liquor of taurine under the temperature-reducing condition, and the last mother liquor removed of impurity is generated after solid-liquor separation; adjust the solution into acid or neutral.

It is preferable to adjust the pH value of the said last mother liquor solution of the taurine after activated carbon treatment with sulfuric acid or other liquor acid to 2.5-7.0; more preferably, the said liquor acid shall not be used to adjust the pH value, but before solid-liquor separation or after separation, the pH value of the last mother liquor of taurine solution is adjusted by a cation exchange resin, and the pH value is adjusted to 3.0-6.0, preferably 3.0-3.5.

Preferably, the said temperature lowering condition means that the temperature of the production system is lower than the temperature of the previous process, and the system processing temperature is controlled between 15-25°C; more preferably 18-22°C;

Based on the said inventive concept, applying the said method for removing the impurity and recovering the mother liquor to the ethylene oxide taurine production process, including the following steps:

S1: ethylene oxide reacts with sodium bisulfite solution to generate sodium isethionate;

S2: mix the sodium isethionate generated in S1, the treated mother liquor, and ammonia water to generate the reaction solution, and then the ammonia is absorbed to a certain concentration, perform an ammonialysis reaction under the action of a catalyst, and evaporate to generate a sodium taurine solution;

S3: the sodium taurine solution generated in S2 is prepared to a certain concentration, and then is treated with acidic cation exchange resin to generate liquor of taurine, which is then concentrated and crystallized to generate the crude taurine and mother liquor; or by adding sulfuric acid to reach the pH value of 7.0-8.5 to generate taurine crystal liquor, after cooling and crystallization, crude taurine and mother liquor are generated;

S4: after the mother liquor collected in S3 is concentrated and crystallized for multiple times, the taurine is separated and extracted through filtering equipment such as the plate and frame, and is concentrated to generate the last mother liquor of taurine;

S5: the last mother liquor of taurine collected in S4 is cooled to 15-25 ° C, then a certain amount of activated carbon is added, and sulfuric acid is added to adjust the pH value to 2.5-7.0, and the mother liquor is separated through filtering equipment such as the plate and frame and the microporous filter; as an option, instead of adding the said sulfuric acid to adjust the pH value, acid reduction treatment is performed by the acidic cation exchange resin before or after solid-liquor separation;

S6: the mother liquor collected by S5 is passed through the alkali anion exchange resin, and the anion exchange resin exchanges with taurine, sodium isethionate, etc., and the effective ingredients are absorbed on the resin, and there is the liquor with more impurities at the collection outlet, which needs subsequent processing; after collection to a certain degree, the resin is regenerated with an alkaline solution to separate impurities from effective ingredients, and the outlet material liquor is collected as the taurine mother liquor after removing impurities;

S7: pass the taurine mother liquor collected in S6 into liquor ammonia or ammonia gas under the cooling condition until the mass-volume ratio of ammonia content is greater than 15g/100ml, a large amount of sulfate and the other impurities will be precipitated, and then a leaf filter could be used or it can be filtered in the closed plate and frame to generate the clear mother liquor, the mother liquor generated can be returned to step S2 and used in the ammonia hydrolysis step.

It should be noted that, among them, the sodium isethionate produced by S1 can be concentrated and crystallized and dried to generate the correspondent solid, or after the direct reaction is completed, the mixed liquor generated by the reaction is directly mixed with the ammonia water of S2 to generate the corresponding solid. The reaction solution has a concentration of ammonia of 20-28 wt.% (weight percent) in the reaction solution after ammonia absorption.

Specifically, the concentration of the sodium bisulfite solution in S1 is 9-36 wt%, and the ratio of the amount of the sodium bisulfite to the amount of the ethylene oxide substance is 1: 0.95-1.

Specifically, the catalyst in S2 is any one or the mixture of any two or more of sodium hydroxide, potassium hydroxide, lithium hydroxide, sodium carbonate, potassium carbonate, and lithium carbonate, and the temperature of the ammonolysis reaction is 150 -290 °C, and pressure is 10-25MPa.

Specifically, when the acid cation exchange resin column treatment is used to treat sodium taurine in S3, the concentration of the sodium taurine solution is 15% -35%, preferably 18% -20%. In S3, when sulfuric acid is used to treat sodium taurine, the concentration of the sodium taurine solution is 25% -40%, preferably 32% -38%.

The crude taurine in S3 needs to be dissolved by adding water, activated carbon, etc., and then decolorized, filtered, cooled, crystallized, and dried after centrifugation to generate the finished taurine product. The refined mother liquor after centrifugation can be reused to prepare the sodium taurine solution or sent to cattle crude sulfonic acid in decolorizing ingredients.

Specifically, the mother liquor in S4 is concentrated and crystallized for multiple times and concentrated once or twice to respectively generate the crude product twice or three times. The last mother liquor of the generated taurine may be the secondary mother liquor or the third mother liquor. In S3, when acid cation exchange resin column treatment is used to treat sodium taurine, the last mother liquor of taurine is preferably the second mother liquor; when S3 is used in the treatment of sodium taurine, the last mother liquor of taurine is the third mother liquors preferably.

Based on the said removing impurity and recovery method, this invention provides a set of removing impurity and recovery system for processing the last mother liquor of taurine in the ethylene oxide process of taurine production process, including the anion resin adsorption device and the ammonia mixing and desalination device connected successively to adsorb the effective components. The feed port of the said anion resin adsorption device is connected to the outlet of the last mother liquor of taurine, and the last mother liquor of taurine is generated in the previous step. The said anion resin adsorption device is provided with an anion exchange resin column. The said ammonia mixing and desalination device includes an ammonia mixing reaction tank provided with the circulation path and the sealed filtering device.

Preferably, the activated carbon decolorization and removing impurity device is provided between the said anion resin adsorption device and the discharge port of the last mother liquor of taurine. The said activated carbon decolorization and removing impurity device includes a decolorization tank and a filtration device.

Preferably, the front end or the rear end of the said activated carbon decolorization and removing impurity device is connected to the cationic resin adsorption device, and the said cationic resin adsorption device is used to reduce the pH value of the last mother liquor of taurine.

Preferably, when the said rear end of the activated carbon decolorization and removing impurity device is connected to a cationic resin adsorption device, the said cationic resin adsorption device includes a raw material tank and a cation exchange resin column, and the said raw material tank and the said output of the activated carbon decolorization and removing impurity device are connected. The said cation exchange resin column and the said anion exchange resin column are connected.

Preferably, the said anion exchange resin column controls the addition of alkaline liquor through a regeneration feed valve and absorbs the anions on the said anion exchange resin column, and the absorbed anions are eluted and collected in a mother liquor receiving tank through a regeneration manner; the outlet of the said mother liquor receiving tank is connected to the feed port of the said ammonia mixing and desalination device.

Preferably, the said ammonia mixing reaction tank is provided with an ammonia inlet, a feed inlet and a discharge outlet, and the outlet of the said ammonia mixing reaction tank is connected to the feed inlet of the said sealed filtering device through a pump, and a transfer discharge valve is provided with the said pump to discharge the filtered clear material.

Preferably, the said anion resin adsorption device includes a raw material tank, a raw material pump, an anion exchange resin column, and a receiving tank which are sequentially connected, wherein the raw material tank and the receiving tank are both atmospheric pressure devices.

Preferably, the said anion exchange resin column is an alkali anion exchange resin column.

Preferably, the said sealed filtering device is a leaf filter or a sealed plate and frame filter.

Preferably, the said activated carbon decolorization and removing impurity device includes a decolorization tank, a primary filtration device, a transit tank, a secondary filtration device, and a receiving storage tank that are connected in sequence. The said decolorization tank is provided with a temperature reduction mechanism, and the said secondary filtration device is a precision filter.

Preferably, the temperature reduction mechanism is a water circulation condensing layer provided outside the decolorizing tank, and the cooling water inlet and outlet valves control the inflow and outflow of the condensed water.

Preferably, the said primary filtering device is a plate and frame filter or a microporous filter.

Compared with the current technology, this invention has the following advantages and beneficial effects:

1. The method and system used in this invention, after treating the last mother liquor of taurine with an anion exchange resin, can remove more impurities in the taurine mother liquor, and remove the salt further by ammonia mixing treatment to generate the pure taurine mother liquor, thus realizing the increase of recycling of mother liquor and increase the product yield.

2. The activated carbon decolorization and removing impurity device used in this invention, under cooling conditions, uses activated carbon to adsorb a certain amount of impurities such as ethylene glycol, metal ions, and a small amount of organic matter, so that the impurities in the taurine mother liquor can be removed more thoroughly.

3. The removing impurity and recovery system of this invention adopts an optimized design and uses the removing impurity device to remove various impurities contained in the last mother liquor through a targeted manner, which is efficient and thorough, simple in operation, and low in operation costs.

## Brief Description of the Drawings

In order to explain the Embodiment's technical solution of this invention more clearly, the drawings used in the description of the Embodiments are briefly introduced one by one, obviously, the drawings in the following description are some Embodiments of this invention, the ordinary technicians in this technical field can generate the other drawings based on these drawings without paying creative labor.

Figure 1 is the process flow chart of removing impurity and recycling the mother liquor in the Embodiment 1 of this invention.

Figure 2 is the process flow chart of the mother liquor for removing impurity and recovery of Embodiment 2 of this invention.

Figure 3 is the schematic diagram of the mother liquor removing impurity and recovery system in Embodiment 1 of this invention.

Figure 4 is the schematic diagram of the mother liquor removing impurity and recovery system in Embodiment 2 of this invention.

Figure 5 is the schematic diagram of the mother liquor removing impurity and recovery system in Embodiment 3 of this invention.

Figure 6 is the schematic diagram of the mother liquor removing impurity and recovery system in Embodiment 4 of this invention.

Figure 7 is the process flow chart of the ethylene oxide process of taurine production process (cation exchange process) provided by this invention.

Figure 8 is the process flow chart of the ethylene oxide process of taurine production process (sulfuric acid neutralization process) provided by this invention.

## Detailed Description

The following content describes the specific content of this invention in detail with the attached drawings and specific Embodiments, the examples given are only used to explain this invention and are not intended to limit the scope of the present invention.

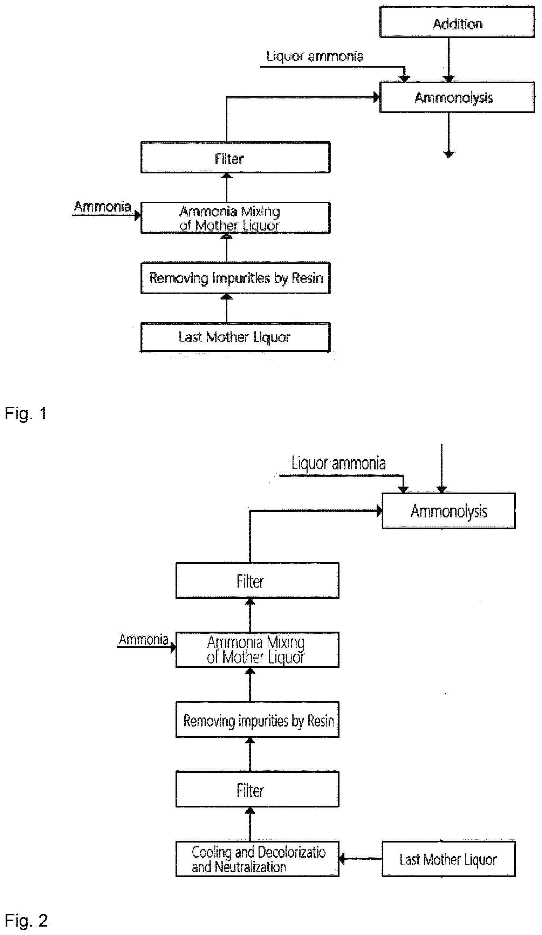

As shown in Figure 1, this invention provides a method for removing impurities from the mother liquor of taurine, which is applied to the ethylene oxide process for preparing taurine, and passing the last mother liquor of taurine obtained by multiple concentration and crystallization in the production process through anion exchange resin to be ion exchanged; then the alkaline solution is used to elute and regenerate the anion exchange resin to collect the outlet material liquor; the collected outlet material liquor is subjected to ammonia mixing treatment, and solid-liquor filtration separation to generate the impurity-removed mother liquor; the mother liquor enters the ammonolysis step, which is added as a raw material.

As shown in Figure 2, this invention provides a preferred method for removing impurities and recovering taurine mother liquor, a step is added to the process of the said Embodiment, that is, the activated carbon decolorization and removing impurity. This process is preferably the first step of removing impurities, that is, lowering down the temperature of the last mother liquor of taurine at first, and then decolorization and removing impurities by activated carbon, solid-liquor filtration and separation, and then the process of anion resin adsorption is performed to further remove impurities, after filtration, the ammonia mixing treatment removes the sulfate in the mother liquor, and the removing impurity process of the mother liquor is completed, and it shall be returned to the ammonia hydrolysis process to join the cycle production.

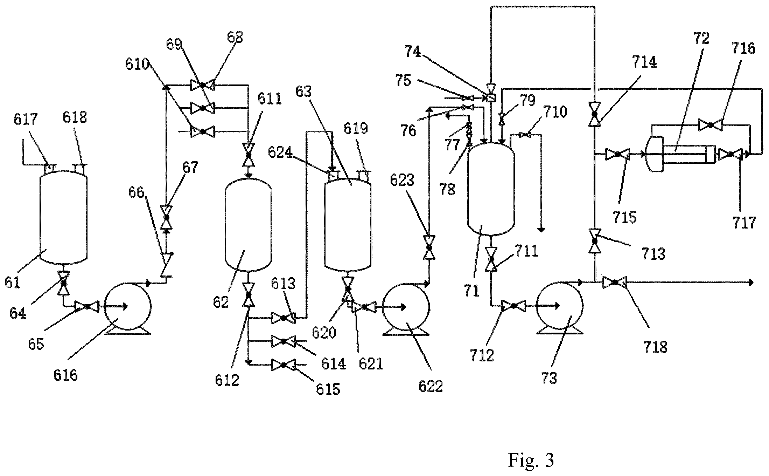

As shown in Figure 3, this invention provides a taurine mother liquor removing impurity and recovery system, utilizing the removing impurity and recovery method shown in Figure. 1, which includes an anion resin adsorption device and an ammonia mixing and desalination device connected sequentially.

Among them, the feed port 617 of the anion adsorption device is connected to the outlet of the last mother liquor of taurine generated in the previous step. The anion resin adsorption device includes raw material tank 61, anion exchange resin column 62, and receiving tank 63, raw material pump 616 is connected between raw material tank 61 and anion exchange resin column 62, and both raw material tank 61 and receiving tank 63 are atmospheric pressure equipment.

Above raw material tank 61 is provided with mother liquor feed port 617 and exhaust port 618, and discharge valve 64 is provided below; raw material pump 616 is provided with feed valve 65, discharge check valve 66 and discharge valve 67; above the column of the anion exchange resin, mother liquor feed valve 68, water washing valve 69 and regeneration feed valve 610 are arranged in parallel, the liquid in the three valves is controlled to enter the anion exchange resin column 62 by a total feed valve 611, and anion exchange resin column 62 is provided with general discharge valve 612, which controls the regeneration discharge valve 613, washing water discharge valve 614, and material discharge valve 615 arranged in parallel, inlet port 624 above receiving tank 63 connects with the pipeline of regeneration discharge valve 613, and discharge valve 620 is provided at the bottom, which is connected to inlet valve 621 of transit pump 622, transit pump 622 is provided with transit outlet valve 623, and exhaust port 619 is provided above receiving tank 63. Anion exchange resin column 62 controls the addition of the alkaline liquor through regeneration feed valve 610, and the anion adsorbed on the anion exchange resin column 62 is eluted in a regeneration manner and collected into mother liquor receiving tank 63; the material discharged from mother liquor receiving tank 63 is controlled and transferred to the feed port of the ammonia mixing and desalination device by transfer pump outlet valve 623 of the transfer pump. In addition to the said pipeline valves, the device is also provided with some valves and connection components necessary for production equipment, which are common technical means in the field, and will not be repeated here.

Anion resin adsorption device can remove most of the impurities in the last mother liquor, and the operation process is simple. During operation, the last mother liquor material is added to raw material tank 61 through raw material tank feed port 617, and open raw material tank discharge valve 64, raw material pump feed valve 65, raw material pump discharge valve 67, and anion exchange resin column raw material feed valve 68, anion exchange resin column feed valve 611, anion exchange resin column discharge valve 612, anion exchange resin column material discharge valve 615, start resin column raw material feed pump 616 and wait for the material to be discharged from material discharge valve 615 of anion exchange resin column, observe the pH value at outlet and detect the content of the outlet material, when the adsorption is saturated, stop resin column raw material feed pump 616, close anion exchange resin column raw material feed valve 68 and anion exchange resin column material discharge valve 615. Open anion exchange resin column washing water discharge valve 614 and anion exchange resin column water washing valve 69, and after cleaning for the pre-set time, close anion exchange resin column washing water discharge valve 614 and anion exchange resin column water washing valve 69; open anion resin column regeneration discharge valve 613 and anion exchange resin column regeneration feed valve 610, the resin is regenerated with alkali, and the effective components in the mother liquor adsorbed on the resin column are eluted at the same time, and the eluted mother liquor is sent to receiving tank 63; the exhaust port of the atmospheric pressure device is in the status of normal open.

Ammonia mixing and desalination device includes ammonia mixing reaction tank 71, a pump 73, and a leaf filter 72 connected sequentially, the leaf filter 72 can also be replaced by a sealed plate and frame filter. Above ammonia mixing reaction tank 71, set ammonia absorber 74, ammonia inlet valve 75, feed valve 76, clear liquor return tank valve 79, emptying valve 710, safety valve 77 and front safety valve control valve 78, at the bottom of the ammonia mixing reaction tank set discharge valve 711, which is connected to pump feed valve 712, the other end of the pump 73 is provided with pump discharge valve 713 and transit discharge valve 718, the outlet direction of pump discharge valve 713 is connected to ammonia absorber 74 through return valve of inlet vane 714 and connected to leaf filter 72 through inlet valve of the blade machine715, the leaf filter is provided with overflow valve 716 and discharge valve 717; and discharge valve 717 is connected to clear liquor return tank valve 79. Similarly, some valves and connection components necessary for production equipment are also provided in the device, which are common technical means in the field, and will not be repeated here.

The said ammonia mixing and desalination device can remove salts and impurities in the mother liquor, and the operation process is simple. Operation method: open front control valve 78 of safety valve, open emptying valve 710, open feed valve 76 to add mother liquor to ammonia mixing reaction tank 71, and close feed valve 76 after feeding. Open discharge valve 711 at the bottom, pump feed valve 712, and return valve of inlet vane 714, start pump 73, open pump discharge valve 713, open ammonia inlet valve 75 after stabilization, close emptying valve 710, and absorb ammonia until the content of ammonia is more than 15% (mass-volume ratio, 15 g/100 ml), then stop absorption and close ammonia inlet valve 75; open overflow valve of leaf filter 716, clear liquor return tank valve 79, inlet valve of the blade machine 715, after leaf filter 72 is filled, open the leaf filter discharge valve 717, and close overflow valve of leaf filter 716 at the same time. During this cycle, samples were taken for observing the state of the materials in ammonia mixing reaction tank 71 until the filtrate is clear. After filtrate is clear, open transit discharge valve 718 to transfer the material to the ammonolysis reaction process.

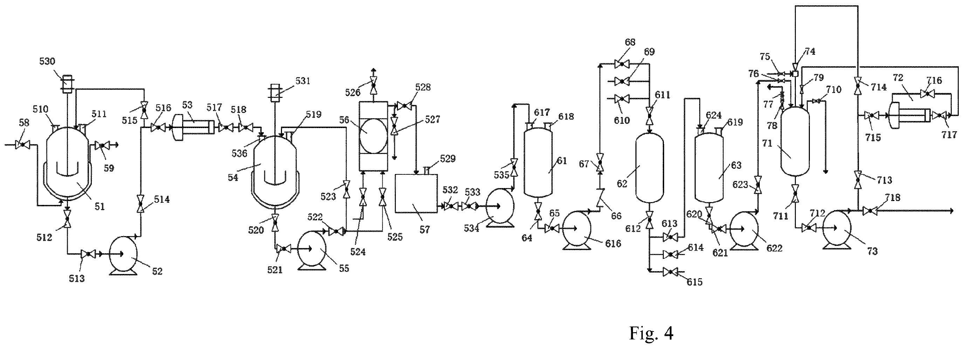

For the said taurine mother liquor removing impurity and recovery method shown in Figure 2 of this invention, the correspondent process equipment connection diagram is shown in figure 4. Compared with the said removing impurity and recovery system, the system is added a set of activated carbon decolorization and removing impurity device in front of the anion resin adsorption device. That is, the last mother liquor of the taurine generated in the previous step is first decolorized and impurity-removed by activated carbon, and then sent to the anion resin adsorption device for adsorption treatment. Specifically, the activated carbon decolorization and removing impurity device includes decolorization tank 51, plate and frame filter pump 52, plate and frame filter 53, relay tank 54, precision filter pump 55, precision filter 56, and receiving tank 57, and transfer pump 534 which are connected sequentially. Compared with plate and frame filter 53, the pore diameter of fine filter 56 is smaller and can filter out small particles of impurities. Among them, decolorizing tank 51, transfer tank 54 and receiving storage tank 57 are all atmospheric pressure equipment.

A water circulation condensation layer is provided on the outside of decolorization tank 51 to lower down the temperature in the tank of decolorization tank 51, the interlayer is provided with cooling water inlet valve 58 and cooling water outlet valve 59, decolorization tank 51 is provided with stirring mechanism 530, mother liquor feed port 510 and exhaust port 511 are provided in the top while the discharge valve 512 is provided at the bottom, which are connected to the plate and frame filter pump 52 through the plate and frame filter pump and feed valve 513, plate and frame filter pump 52 is provided with discharge valve 514 and return valve 515; the inlet and outlet of plate and frame filter 53 are provided with feed valve 516 and discharge valve 517 respectively, and it is connected to transit tank 54 through transit tank feed valve 518, above transit tank 54, set discharge port 536, exhaust port 519 and stirring mechanism 531; and discharge valve 520 at the bottom is connected to precision filter pump feed valve 521, and the outlet of the precision filter pump 55 is connected to discharge valve 522, which is connected to precision filter pump return valve 523 and precision The filter feed valve 525 respectively, above precision filter 56, set inlet cleaning water valve 524, outlet cleaning water valve 527 and exhaust valve 526, discharge valve 528 of precision filter outlet is connected to the inlet of receiving tank 57, receiving tank 57 is provided with exhaust port 529, and its discharge valve 532 is connected to the inlet valve 533 of transit pump 534, and outlet valve 535 of transit pump 534 is connected to raw material tank 61 in the anion resin adsorption device. Valves and connecting components necessary for other production equipment are common technical fields in the art and will not be repeated here. A feed port for adding liquor acid is preferably provided on the decolorization tank to add liquor acid, such as sulfuric acid, thus reducing the pH value of the solution in the tank, which is more conducive to subsequent removing impurity treatment.

After adding the device, the filtration efficiency and processing capacity can be improved through two-stage filtration. Operation method: Activated carbon is added from mother liquor feed port 510, or can be added from other openings, which is not limited here, the plate and frame filter decolorization tank discharge valve 512 are closed, and the material is added to the decolorization tank 51 through decolorization tank feed port 510, open cooling water outlet valve 59 and cooling water inlet valve 58 and pass in the cooling water to lower down temperature, at the same time, operate decolorizing tank stirring mechanism 530 to cool down to the prescribed temperature, and then close cooling water inlet valve 58 and cooling water outlet valve 59. Open decolorization tank discharge valve 512, plate and frame filter pump feed valve 513, plate and frame feed valve 516, plate and frame discharge valve 517, and transit tank feed valve 518, close transit tank discharge valve 520, and start plate and frame filter pump 52, and then adjust the plate and frame pressure through the plate and frame filter pump return valve 515, activated carbon and materials enter plate and frame filter 53 together, then, activated carbon is trapped in filter 53, open the plate and frame , the activated carbon that has absorbed impurities can be discharged. After the material in the transit tank 54 reaches a certain volume, open transit tank discharge valve 520, precision filter pump feed valve 521, precision filter pump return valve 523, precision filter feed valve 525, precision filter exhaust valve 526 and precision filter discharge valve 528, start precision filter pump 55, open precision filter pump discharge valve 522, and after the precision filter exhaust valve 526 discharges, close precision filter exhaust valve 526, then, the feeding pressure of precision filter 56 is adjusted by precision filter pump return valve 523, the material collected by receiving tank 57 is sent to the subsequent processing station, that is, it is sent to raw material tank 61 of the anion resin adsorption device, after it is processed by the anion exchange resin column 62, send it to ammonia mixing reaction tank 71 for ammonia mixing treatment to remove sulfates and other impurities further.

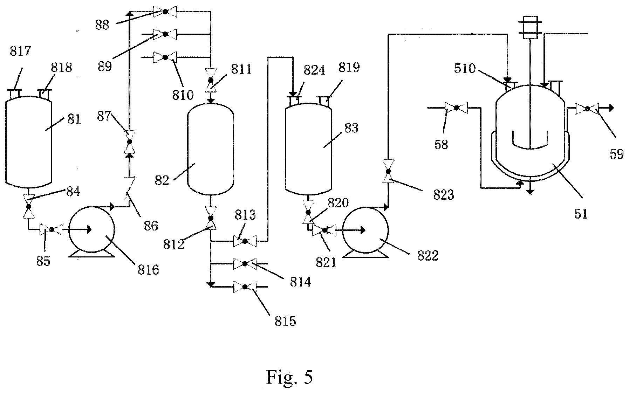

As shown in Figure 5, this invention optimizes further the removing impurity and recovery system, instead of adding liquor acid to the decolorization tank to lower down pH value, the last mother liquor of the taurine generated in the previous step shall go through acid reduction treatment with solid acid to reduce acid firstly, the treatment device is a set of acidic cation exchange resin adsorption device, the acid cation exchange resin adsorption device is connected to the discharge port of the last mother liquor of the taurine in the previous step.

As shown in the figure, the cationic resin adsorption device includes raw material tank 81, cation exchange resin column 82, and receiving tank 83, a raw material pump 816 is connected between the raw material tank 81 and the cation exchange resin column 82, among them, the raw material tank 81 and the receiving tank 83 are atmospheric pressure equipment.

Above raw material tank 81, set mother liquor feeding port 817 and an exhaust port 818; under raw material tank 81, set discharging valve 84; the raw material pump is provided with feed valve 85, discharge check valve 86 and discharge valve 87; above cation exchange resin column 82, there is mother liquor feed valve 88, water washing valve 89 and regeneration feed valve 810, the liquor in the three valves is controlled by total feed valve 811 and enters the cation exchange resin column 82, cation exchange resin column 82 is provided also, and general discharge valve 812, material discharge valve 813, washing water discharge valve 814, and regeneration discharge valve 815 is provided for cation exchange resin column 82, and controlled by general discharge valve 812, and they are arranged in parallel. Inlet 824 above the receiving tank connects with the pipeline of material discharge valve 813, discharge valve 820 is set below and is connected to inlet valve 821 of transit pump 822, the transit pump is provided with transit outlet valve 823, exhaust port 819 is provided above the receiving tank 83. Cation exchange resin column 82 controls the addition of the acidic liquor through the regeneration feed valve 810, and the metal ions and the like adsorbed on the cation exchange resin column 82 are eluted and removed in a regeneration manner. The material is controlled to be transferred to feed inlet of the activated carbon 510 removing impurity device through transit valve 823 of transit pump. In addition to the pipeline valves described above, the device is also provided with some valves and connection components necessary for production equipment, which are common technical means in this field, and will not be repeated here.

The cationic resin adsorption device can remove impurities such as metal ions in the mother liquor and can reduce the pH of the mother liquor without introducing other anions or impurities, and the operation process is simple. Add the last mother liquor of taurine produced in the previous step to raw material tank 81 through raw material tank feed port 817, and open raw material tank discharge valve 84, raw material pump feed valve 85, raw material pump discharge valve 87, and feed valve of raw material of the cationic resin column 88, feed valve of cationic resin column 811, cationic resin column discharge valve 812, cationic resin column material discharge valve 813, start the resin column raw material feed pump 816, and wait for the material to be discharged from the cationic resin column material discharge valve 813 to receiving tank 83, and observe the pH value at the outlet, and detect the content of the material at the outlet, when the pH at outlet or the pH at receiving tank 83 meet the requirement, the resin column raw material feed pump 816 stops, and close cationic resin column raw material feed valve 88 and cationic resin column material discharge valve 813; open cationic resin column water discharge valve 814 and cationic resin column water wash valve 89, and after cleaning for a prescribed time, close cationic resin column wash water discharge valve 814 and cationic resin column water wash valve 89; open cationic resin column regeneration discharge valve 815 and the cationic resin column regeneration feed valve 810, and regenerate the cationic resin column with acid, metal ions and the other impurities adsorbed in the mother liquor on the cation exchange resin column is eluted, regenerated discharge valve 815 of ationic resin column will conduct the subsequent treatment. The mother liquor processed in receiving tank 83 is transferred to decolorizing tank 51 by transit pump 822.

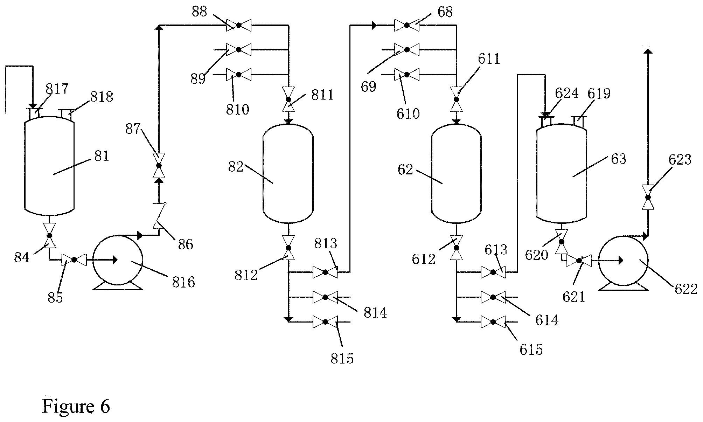

As shown in Figure 6, this invention optimizes the processes and equipment in the said Embodiments. The removing impurity and recovery system is composed of activated carbon decolorization and removing impurity device, the cation exchange resin adsorption device, anion exchange resin adsorption device, the ammonia mixing desalination device. Among them, the cation exchange resin adsorption device and the anion exchange resin adsorption device share a raw material tank and receiving tank. The pH value of the last mother liquor of taurine after treatment is reduced by solid acid. This process involves two chemical reactions, one is the reaction between acidic resin and cation in solution, and the other is the reaction between acid and basic resin. After the stepwise reaction, the required materials are collected, and the separation is carried out to achieve complete separation.

Specifically, raw material tank of cation exchange resin adsorption device 81 is connected to receiving storage tank of the activated carbon decolorization and removing impurity device 57. Raw material pump 816 is connected between raw material tank 81 and cation exchange resin column 82, cation exchange resin column 82 and anion exchange resin column 62 is connected, and anion exchange resin column 62 is connected to receiving tank 63, receiving tank 63 is controlled to be fed to the feed port of the ammonia mixing and desalination device through transit outlet valve 623 of transfer pump 622.

Among them, above raw material tank 81 is provided with mother liquor feed port 817 and an exhaust port 818, and discharge valve 84 is provided below; the raw material pump is provided with feed valve 85, discharge check valve 86 and discharge valve 87; above cation exchange resin column 82, set mother liquor feed valve 88, water washing valve 89 and regeneration feed valve 810, the liquor in these three valves is controlled by general feed valve 811 to enter the cation exchange resin column 82, the cation exchange resin column 82 is provided with general discharge valve 812, control material discharge valve 813, washing water discharge valve 814, and regeneration discharge valve 815, the material discharge valve 813, washing water discharge valve 814, and regeneration discharge valve 815 are arranged in parallel., the feed port above the anion exchange resin column connects with the pipeline of material discharge valve 813 and is controlled by mother liquor feed valve 68, water washing valve 69 and regeneration feed valve 610 are also set above the anion exchange resin column, the liquor in the three valves is controlled by general feed valve 611 to enter the anion exchange resin column 62, the anion exchange resin column 62 is provided with general discharge valve 612, which controls regeneration discharge valve 613, washing water discharge valve 614, and material discharge valve 615, the regeneration discharge valve 613, washing water discharge valve 614, and material discharge valve 615 are arranged in parallel. Inlet 624 above the receiving tank 63 connects with the pipeline of regeneration discharge valve 613, discharge valve 620 is provided below receiving tank 63 and is connected to inlet valve 621 of transit pump 622, and transit pump is provided with transfer outlet valve 623. Anion exchange resin column 62 controls the addition of alkaline liquor through regeneration feed valve 610, and the anion adsorbed on the anion exchange resin column 62 is eluted in a regeneration manner, and then collected to the mother liquor receiving tank 63; the discharge material of the mother liquor receiving tank 63 is controlled to be transferred to the feed port of the ammonia mixing and desalination device through the transit pump outlet valve 623 of the transfer pump.

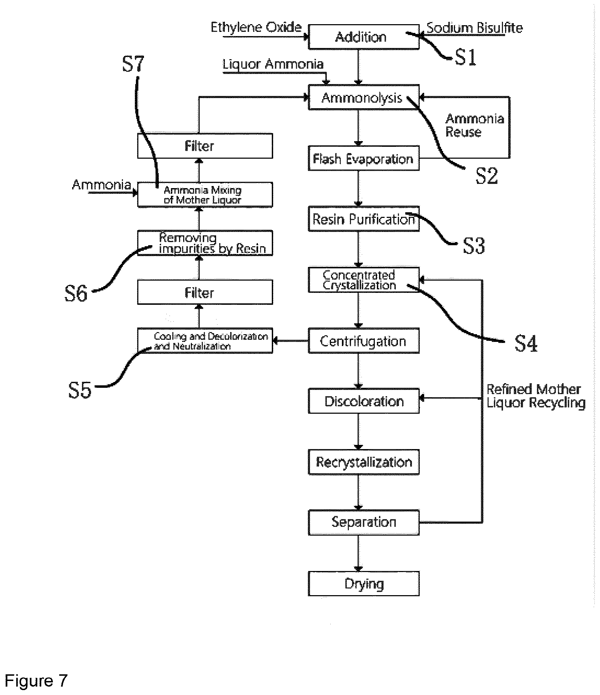

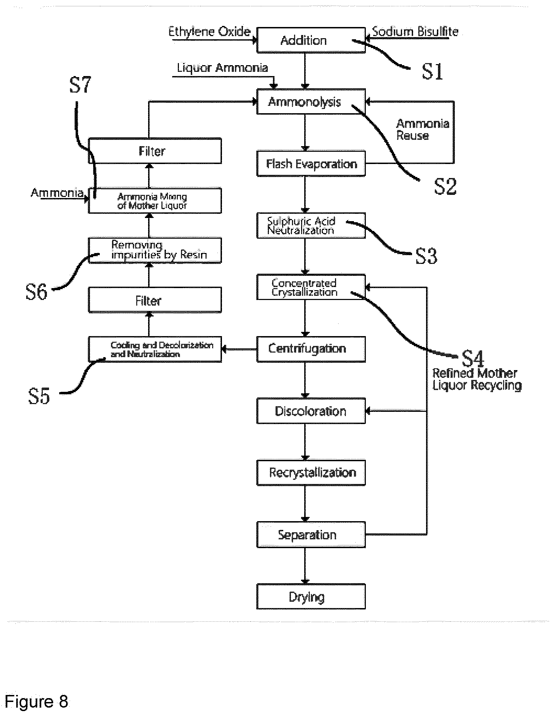

As shown in Figure 7 and Figure 8, this invention provides two processes for producing taurine by the ethylene oxide process, and the said method and device for removing impurities are applied. The difference between the two methods lies in different post-ammoniolysis processes, one uses cationic resin process, about this process, the applicant's other Chinese invention patent CN107056659 has a detailed record, the other method uses a sulfuric acid neutralization process. In the following Embodiments, each step in the taurine mother liquor removing impurity and recovery method provided by this invention is decomposed, and each embodiment is a step or part of a step in the method. The two processes shown in Figure 7 and Figure 8 are combined below to illustrate the taurine production process.

S1. Ethylene oxide is reacted with sodium bisulfite solution to generate sodium isethionate; in this step, impurities such as ethylene glycol and polyethylene glycol are generated.

S2. The reaction solution is obtained by mixing sodium isethionate obtained from S1, mother liquor after the removing impurity treatment and ammonia water, absorbing ammonia to a certain concentration, and ammonolysis is carried out under the action of catalyst, after the reaction is completed, flash evaporation process is performed to remove the excess ammonia gas from the reaction solution to be recycled as the raw material for the ammonolysis reaction, and then evaporated to generate a sodium taurine solution; the ethylene glycol by-products in the sodium isethionate solution in step S1 are converted into organic impurities such as polyether alcohols. Because the ammonolysis reaction is reversible, according to the theory of chemical equilibrium, the presence of sodium ditaurate and sodium tritaurate can achieve the purpose of improving the conversion of raw materials. Recycling the mother liquor after the removing impurity treatment can achieve the purpose of increasing the application of liquor and reducing the side reaction, and further achieve the purpose of further stabilizing production and improving yield.

S3. The sodium taurine solution generated in S2 is prepared to a certain concentration, the raw material liquor of taurine can be generated by passing it through acidic cation exchange resin column, and the crude taurine and mother liquor can be generated by concentration and crystallization, the separation temperature is about 15 -25°C; or use sulfuric acid neutralization process, by adding sulfuric acid until pH value is 7.0-8.5, to generate taurine crystal liquor. After cooling and crystallization, crude taurine and mother liquor shall be generated, and the separation temperature is about 32-35°C.

S4. The mother liquor collected in S3 is further concentrated and crystallized for multiple times, and taurine is separated and extracted through equipment such as plate and frame, and then the last mother liquor of concentrated taurine is generated.

S5. First, the last mother liquor of taurine collected in S4 is cooled to 15-25°C to generate the best crystallization effect and precipitate impurities. Then, a certain amount of activated carbon is added, filter the solid impurities through equipment such as plate frame and microporous filter to generate the mother liquor after removing impurities.; in this step, the pH value of the solution needs to be adjusted to be acidic or neutral., two methods can be used, one method is to add liquor acid, such as sulfuric acid, to adjust the pH value of the solution to 2.5-7.0, another way is to treat the last mother liquor of taurine with an acidic cation exchange resin to adjust the pH value to 3.0-6.0.

S6. Pass the mother liquor collected by S5 through the alkali anion exchange resin column, the material liquor at the collection outlet contains a lot of impurities and needs subsequent treatment; after collecting to a certain extent, the alkaline solution, such as liquor alkali, is used to regenerate the resin column, the material liquor at collection outlet is the mother liquor containing taurine that is thoroughly decontaminated. The pH value of the solution is controlled so that most of the taurine in the solution exists in form of anion, according to the principle of anion resin adsorption, the resin exchanges with the taurine anion and adsorbs the taurine anion on the resin, at the same time, sodium isethionate, sodium ditaurate, and sodium tritaurate in the mother liquor also exist in form of anion, can also exchange with the resin, and are adsorbed in the resin. Organic impurities such as ethylene glycol and polyether alcohol do not exchange with the resin and cannot be adsorbed by the resin, taurine and sodium isethionate adsorbed on the resin are eluted through an alkaline solution to generate pure cattle sulfuric acid mother liquor, so as to achieve the separation of effective ingredients and impurities, to fulfill the purpose of mother liquor removing impurities.

S7. Pass the thoroughly decontaminated taurine-containing mother liquor collected in S6 into liquor ammonia under cooling conditions, and until the ammonia content is greater than 15%, a large amount of sulfate and other impurities will be precipitated, subsequently, a clear mother liquor is filtered by means of leaf filter or closed plate frame, and the mother liquor generated can be used in the ammonia hydrolysis step (Step S2). The said filtering device needs to meet environmental protection requirements and must be sealed to prevent ammonia leakage.

The process principle of the cation exchange resin in Step S5 is explained below:

The acidic cation exchange resin in step S5 can be regarded as solid acid. The chemical reactions that occur with cation exchange resins are as follows (cations are only represented by $Na^+$, and regeneration acids are only represented by $H_2SO_4$):

$$RH + Na^+ \rightarrow RNa + H^+ \qquad (1)$$

$$2RNa + H_2SO_4 \rightarrow 2RH + Na_2SO_4 \qquad (2)$$

Among them, RH indicates that the acidic cation exchange resin is in a hydrogen state (that is, a state where regeneration is completed), and RNa indicates that the acidic cation exchange resin is in a sodium-absorbing state (that is, a state after being saturated). The principle of regenerated cation exchange resin is that the cations in the solution have affinity with the groups opposite to the electricity on the resin, so the cations in the solution remain on the resin, and the hydrogen ions dissociated in the resin are released to the solution and combine with the anions in the solution , the reaction solution can be fully exchanged with the cationic resin to achieve the purpose of lowering the pH value of the solution, when the adsorption is to a certain extent, the pH value of the solution reaches the lowest. After the cation exchange resin is saturated, it needs to be more acidic (that is, the acidity of $H_2SO_4$ in chemical Formula (2) is stronger than that of RH). The regeneration solution exchanges the cations remaining on the resin again to generate RH. At the same time, the regeneration $Na_2SO_4$ will be separated out as a solution. In this way, RH can generate $H^+$ again, that is, it can continue to be used by chemical Formula (1).

In order to illustrate the technical effects of the present invention, the following examples are used for explanation. Unless otherwise specified, the raw materials used in the following examples are all spot goods; unless otherwise specified, the methods used are conventional methods; unless otherwise specified, the material contents refer to mass-volume percentages.

**Embodiment 1**

The content shown in this example is a method for processing an anion exchange resin column in step S6:
At the beginning of pretreatment, the anion exchange resin is loaded into the exchange column by wet method, and the flow rate is 1.8-2.2 BV/h (any value) in the forward direction with 2BV, 4% sodium hydroxide solution (mass percentage, the same below). Pass the resin in the exchange column, and then use deionized water to wash the acidity to pH value = 9 or so. When packing the column, add the resin firstly to the measuring cylinder by wet method, the upper layer of the resin guarantees a water layer of about 5-10cm, shock the graduated cylinder slightly to make the resin dense, and then transfer it into the exchange column by wet method, a 5-10cm water layer should be left on the upper part of the resin layer, and no air bubbles should be left in the resin layer. During the regeneration process, there is no column loading step, but water is directly added to drain the material from the resin column, and then 2BV, 4% sodium hydroxide aqueous solution is passed through the resin in the exchange column in the forward direction at a flow rate of 2BV / h, and the like, after charging 1 BV, the regeneration eluate is collected, then, using deionized water, washing the acidity to pH value = 9 or so, the regeneration is completed, the washing water can be mixed with the regenerated eluent, and the regenerated eluate and the eluate mixed with the washing water can be used as the mother liquor containing taurine for subsequent reprocessing and reused in the ammonolysis reaction.

**Embodiment 2**

The content shown in this Embodiment is that the taurine mother liquor generated after the removing impurity treatment of the last mother liquor of taurine according to the Step S5 and Step S6:

(1) Taurine mother liquor treatment: Take 1000ml of taurine mother liquor, the mass volume percentage is 10% (based on taurine, 100mL of solution contains 10g of taurine), lower down temperature to 15-25°C, add 1g of activated carbon, add sulfuric acid, adjust the pH to 3-3.5, and suction-filter to generate 950 ml of mother liquor.

(2) Take 500ml of the above mother liquor and pass it forward through the resin column at a flow rate of 0.25-2.5BV/h (any value), initially, comes the water at the outlet from the resin layer, and it may not be collected (you can determine whether the material solution comes out by detecting the pH, and the material solution flows out when the pH changes), after the material liquor comes out, start metering and collection, and sample every 0.08-0.15BV (any value) to detect the pH value, when the outlet material liquor index is basically the same as the liquor inlet index (that is, the pH is close), stop feeding; then, use the deionized water to wash out the material liquor for recovery in a forward direction at a flow rate of 2BV / h again.; after the materials are driven out, 2BV, 4% sodium hydroxide solution is passed in a forward direction through the resin layer at a flow rate of 2BV/h, and the total volume of the collected material is about 820ml, the main components are taurine and sodium isethionate, the taurine content is 6% and the sodium isethionate content is 7.3%.

The testing data is as follows:

| Item | The last mother liquor of taurine (before treatment) | The last mother liquor of taurine (after treatment) |
| --- | --- | --- |
| Ethylene glycol | 6% | 0.5% |
| Fe | 10ppm | <1ppm |

(continued)

| Item | The last mother liquor of taurine (before treatment) | The last mother liquor of taurine (after treatment) |
|---|---|---|
| Content of Taurine | 10% | 6% |
| Content of Sodium isethionate | 12% | 7.3% |
| Outer Appearance | Yellow | Light Yellow |

**Embodiment 3**

The mother liquor treated in Embodiment 2 is subjected to ammonia mixing treatment, as Step S7, and recycled.

Ammonia mixing treatment for recovery of mother liquor: The mother liquor collected in Embodiment 2 is passed through ammonia until its ammonia content reaches 15% -20%, and filtered to generate the transparent and clear solution.

Preparation of sodium taurine: the sodium hydroxide exists as catalyst, and the ammonolysis reaction is performed in the sodium isethionate solution (produced by the enterprise), the mother liquor treated with ammonia mixing, and ammonia gas, at 220-280°C and 10-15 MPa, the ammonolysis reaction is performed for 1 hour., after the reaction is completed, the solution generated after the ammonia gas is removed by flash evaporation is the sodium taurine solution.

After testing, the treated mother liquor is essentially free of sulfate.

**Embodiment 4**

The mother liquor before treated in Embodiment 2 is subjected to ammonia mixing treatment, as Step S7, and recycled.

Ammonia mixing treatment for recovery of mother liquor: The raw material (that is, the mother liquor before treatment) collected in Embodiment 2 is passed through ammonia until its ammonia content reaches 15% -20%, and filtered to generate the transparent and clear solution.

Preparation of sodium taurine: the sodium hydroxide exists as catalyst, and the ammonolysis reaction is performed in the sodium isethionate solution (produced by the enterprise), the mother liquor treated with ammonia mixing and ammonia gas. At 220-280°C and 10-15 MPa, the ammonolysis reaction is performed for 1 hour, after the reaction is completed, the solution generated after the ammonia gas is removed by flash evaporation is the sodium taurine solution.

**Embodiment 5**

Two groups of Embodiments and corresponding comparative embodiments were selected to show the conditions of the ammonolysis reaction and the subsequent extraction of the crude taurine content under various mother liquor reuse conditions;

In the following examples, 1.5 moles of sodium isethionate are used, and the sodium taurine solution is prepared according to the methods of Embodiment 3 and Embodiment 4, the generated sodium taurine solution is treated with cation exchange resin to generate a taurine solution, and then the taurine solution is concentrated and cooled to crystallize to generate a crude taurine product, and the taurine content is measured.

Calculate the yield according to the following formula:

| SN of Experiment | Item | Mother Liquor | | | Sodium isethionate | Sodium taurine solution | The yield of ammonolysis reaction | Crude taurine | |
|---|---|---|---|---|---|---|---|---|---|
| | | Volume (ml) | Taurine content (g/ml) | Sodium isethionate content (g/ml) | Mass (g) | The mass of pure taurine (g) | Calculating by the sodium isethionate input | Mass (g) | Content (g/g) |
| 1 | Prior to treatment of Embodiment 4 | 115 | 10% | 12% | 222 | 172.5 | 92.00% | 162.7 | 88% |
| 2 | Prior to treatment of Embodiment 4 | 135 | 10% | 12% | 222 | 177.2 | 94.50% | 171 | 86% |
| 3 | After treatment of Embodiment 3 | 190 | 6% | 7.30% | 222 | 187.5 | 100.00% | 173.2 | 92% |
| 4 | After treatment of Embodiment 3 | 225 | 6% | 7.30% | 222 | 206.3 | 110.00% | 188.5 | 93% |

Thereinto, the yield of ammonolysis reaction = the mass of pure taurine : (Mass of sodium

isethionate:$148 \times 125$) $\times 100\%$.

This comparative example shows that in the ammonolysis process, the taurine content in the crude taurine product can reach 92% or more when the last mother liquor of taurine treated in Step S5 and Step S6 is used for production.

By comparing Experiment 1 and Experiment 2 in Embodiment 5, the increase in the application of the mother liquor before treatment will increase the yield of the ammonolysis reaction; but at the same time, the crude content will decrease, which will eventually affect the quality of the finished product, which means that the untreated mother liquor can only apply to a certain extent, and it is why the amount of mother liquor in the alkylene oxide process route cannot continue to increase.

By comparing Experiment 1 and Experiment 3, Experiment 2 and Experiment 4 in Embodiment 5, the amount of the mother liquor before and after the treatment is the same (the same mass of pure), the yield of the ammonolysis reaction is significantly increased, and the content of crude products increases significantly, this fully demonstrates that the mother liquor is removed of impurities more thoroughly after the treatment, which causes the side reactions under the conditions of the ammonolysis reaction to be greatly reduced and the product quality to be greatly improved.

By comprehensively comparing Experiments 1, 2, 3, and 4 in Embodiment 5, the effect of the mother liquor after removing impurity treatment on improving the yield and the crude product content is very obvious, at the same time, the application of the mother liquor after the treatment can obviously increase the dosage for use.

**Embodiment 6**

The content shown in this Embodiment is the taurine mother liquor generated after the removing impurity treatment of the last mother liquor of taurine according to Step S5 and Step S6;

(1) Taurine mother liquor treatment: Take 1000ml of taurine mother liquor, in which the mass volume percentage is 10% (based on taurine, 100mL of the solution contains 10g of taurine). Lower down the temperature to 15-25°C, then add 1g of activated carbon, adjust the solution pH to 3-3.5 by cation exchange resin treatment method, and then concentrate and reduce the mother liquor to 950ml.

(2) Take 500ml of the above mother liquor and pass it through the anion exchange resin column in a forward direction at a flow rate of 0.25-2.5BV/h (any value), the initial moisture at the outlet comes from the resin layer, which is unnecessary to be collected. (It can be judged whether the material liquor flows out by detecting the pH Value, when the pH value changes, it indicates that the material liquor flows out.), after the material liquor comes out, start to measure and collect, and sample every 0.08-0.15BV (any value) to detect the pH value, when the material index at the outlet is basically the same as the liquor intake index (that is, the pH values are close), stop feeding; then use deionized water to clean and recover the material liquor in a forward direction at a flow rate of 2BV/h; wait until the material is exhausted, and then use the 2BV, 4% sodium hydroxide solution passes through the resin layer in a forward direction at a flow rate of 2BV/h, the total volume of the collected materials is about 820ml, the main components are taurine and sodium isethionate, the taurine content is 6.3% and the sodium isethionate content is 7.5%.

The testing data is as follows:

| Item | The last mother liquor of taurine (before treatment) | The last mother liquor of taurine (after treatment) |
| --- | --- | --- |
| Ethylene glycol | 6% | 0.5% |
| Fe | 10ppm | <0.5ppm |
| Content of Taurine | 10% | 6.3% |
| Content of Sodium isethionate | 12% | 7.5% |
| Outer Appearance | Yellow | Colorless and light yellow |

Comparison of Embodiment 2 and Embodiment 6

Before treatment: Taurine: 1000 * 10% = 100g;

Sodium isethionate: 1000 * 12% = 120g;

After treatment of Embodiment 2: taurine 950/500 * 820 * 6% = 93.48g

Sodium isethionate: 950/500 * 820 * 7.3% = 113.73g;

Taurine recovery rate = 93.48 / 100 * 100% = 93.48%

Sodium isethionate Recovery Rate = 113.73 / 120 * 100% = 94.78%

After treatment of Embodiment 6: taurine 950/500 * 820 * 6.3% = 98.15g

Sodium isethionate: 950/500 * 820 * 7.5% = 116.85g;

Taurine recovery rate = 98.15 / 100 * 100% = 98.15%

Sodium isethionate Recovery Rate = 116.85 / 120 * 100% = 97.38%

| Item | Mother liquor | | | Taurine | Sodium isethionate | Recovery Rate | |
|---|---|---|---|---|---|---|---|
| | Volume ml | Taurine content (g / ml) | Sodium isethionate content (g / ml) | Mass (g) | Mass (g) | Taurine (100%) | Sodium isethionate (100%) |
| Before Treatment | 1000 | 10% | 12% | 100.00 | 120.00 | | |
| After Treatment of Embodiment 2 | 1558 | 6% | 7.30% | 93.48 | 113.73 | 93.48% | 94.78% |
| After Treatment of Embodiment 6 | 1558 | 6% | 7.50% | 98.15 | 116.85 | 98.15% | 97.38% |
| Note: The volume of mother liquor after treatment = 950/500 * 820 = 1558ml | | | | | | | |

It is found through experiments that the recovery rate is higher with cationic resin treatment than with sulfuric acid treatment, and the solution color is lighter.

Claims

1. Method for removing impurities out of taurine mother liquor and taurine mother liquor recovery, which is used for the taurine production process in the ethylene oxide process to treat the last mother liquor of taurine, comprising the following steps:

(a) the last mother liquor of taurine is ion-exchanged through anion exchange resin, and the effective components are adsorbed on the anion exchange resin; then the anion exchange resin is eluted and regenerated with alkali solution, and the outlet liquor is collected;
(b) ammonia mixing treatment is performed on the outlet material liquor collected in step (a), and solid-liquor separation is performed to generate mother liquor after removing impurities;

(c) the mother liquor generated in step (b) enters the ammonolysis step.

2. The method according to claim 1, wherein during the said ammonia mixing treatment, liquor ammonia or ammonia gas is added to the said outlet material liquor, and the mass-volume ratio of ammonia is 15g/100ml or more.

3. The method according to claim 2, wherein the said anion exchange resin is an alkali anion exchange resin.

4. The method according to claim 3, wherein before step (a), performing decolorization and removing impurity treatment on the last mother liquor of taurine with activated carbon, comprising:
the activated carbon is added to the last mother liquor of taurine under the temperature-reducing condition, and the last mother liquor removed of impurity is generated after solid-liquor separation; adjust the solution into acid or neutral.

5. The method according to claim 4, wherein the pH of the solution is adjusted according to the liquor acid or the cation exchange resin.

6. The method according to any one of claims 1 to 5, wherein the temperature-reducing condition means that the temperature of the production system is lower than the temperature of the previous step, and the system processing temperature is controlled between 15-25°C.

7. An ethylene oxide taurine production process, including the following steps:

S1: ethylene oxide reacts with sodium bisulfite solution to generate sodium isethionate;
S2: the sodium isethionate generated from S1 and the mother liquor recovered after removing impurities are subjected to an ammonolysis reaction and concentrated by evaporation to generate sodium taurine solution;
S3: the sodium taurine solution generated in S2 is treated with acidic cation exchange resin to generate liquor of taurine, which is then concentrated and crystallized to generate the crude taurine and mother liquor; or taurine crystallization liquor is generated by neutralization with sulfuric acid, crude taurine and mother liquor are generated after cooling and crystallization;
S4: concentrate and crystallize the mother liquor collected in S3 for multiple times, separate and extract taurine, and concentrate to generate the last mother liquor of taurine;
wherein, any one of the methods for removing impurities from taurine mother liquor according to claims 1 to 6 is used to remove impurities out of the last mother liquor in S4, and the mother liquor after removing impurities and recovering is sent to the ammonolysis reaction step of S2.

8. A system for removing impurities and recovering taurine mother liquor for treating the last mother liquor of taurine in the ethylene oxide process of taurine production process, wherein it comprises consecutively an anion resin adsorption device for adsorbing effective ingredients, the ammonia mixing and desalination device, the feed port of the said anion resin adsorption device is connected to the outlet of the last mother liquor of taurine generated in the previous step, the said anion resin adsorption device is provided with an anion exchange resin column, the said ammonia mixing and desalination device includes an ammonia mixing reaction tank provided with a circulation path and a sealed filtering device.

9. The removing impurities system according to claim 8, wherein an activated carbon decolorization and removing impurity device is provided between the said anion resin adsorption device and the outlet of the last mother liquor of taurine, the said activated carbon decolorization and removing impurity device includes a decolorizing tank and a filtering device.

10. The removing impurities system according to claim 9, wherein a cationic resin adsorption device is connected to the front end or the rear end of the said activated carbon decolorization and removing impurity device and the said cationic resin adsorption device is used to reduce the pH value of the last mother liquor of taurine.

11. The removing impurities system according to claim 10, wherein when the sad cationic resin adsorption device is connected to the rear end of the said activated carbon decolorization and removing impurity device, the said cationic resin adsorption device comprises a raw material tank and a cation exchange resin column, the said raw material tank is connected to the discharge port of the said activated carbon decolorization and removing impurity device, and the said cationic exchange resin column is connected to the said anion exchange resin column.

12. The removing impurities system according to any one of claims 8-11, wherein the said anion exchange resin column controls the addition of alkaline liquor through a regeneration feed valve and absorbs the anions on the said anion exchange resin column, and the absorbed anions are eluted and collected in a mother liquor receiving tank through a regeneration manner; the outlet of the said mother liquor receiving tank is connected to the feed port of the said ammonia mixing and desalination device.

13. The removing impurity system according to claim 12, wherein the said ammonia mixing reaction tank is provided with an ammonia inlet, a feed inlet, and a discharge outlet, and the discharge outlet of the said ammonia mixing reaction tank is connected to the feed port of the said sealed filtering device through a pump, and the said pump is provided with the transfer discharge valve for discharging the filtered clear material.


**Patentansprüche**

1. Verfahren zum Entfernen von Verunreinigungen aus Taurin-Mutterlauge und Rückgewinnung von Taurin-Mutterlauge, die für das Taurin-Herstellungsverfahren im Ethylenoxid-Verfahren zur Behandlung der letzten Taurin-Mutterlauge verwendet wird, umfassend die folgenden Schritte:

   (a) die letzte Taurin-Mutterlauge wird durch ein Anionenaustauscherharz ionenausgetauscht, und die wirksamen Bestandteile werden an das Anionenaustauscherharz adsorbiert; dann wird das Anionenaustauscherharz eluiert und mit Alkalilösung regeneriert, und die Auslasslauge wird gesammelt;
   (b) die in Schritt (a) gesammelte Auslassmaterial -Lauge wird einer Ammoniak-Mischbehandlung unterzogen, und es wird eine Feststoff-Flüssigkeits-Trennung durchgeführt, um nach der Entfernung von Verunreinigungen Mutterlauge zu erzeugen;
   (c) die in Schritt b) erzeugte Mutterlauge wird in den Ammonolyse-Schritt überführt.

2. Verfahren nach Anspruch 1, wobei während der Ammoniak-Mischbehandlung Laugen-Ammoniak oder Ammoniak-Gas zu der Auslassmaterial-Lauge hinzugefügt wird und das Massen-Volumen-Verhältnis von Ammoniak 15 g/100 ml oder mehr beträgt.

3. Verfahren nach Anspruch 2, wobei das Anionenaustauscherharz ein Alkali-Anionenaustauscherharz ist.

4. Verfahren nach Anspruch 3, wobei vor Schritt (a) eine Behandlung zur Entfärbung und Entfernung von Verunreinigungen an der letzten Taurin-Mutterlauge mit Aktivkohle durchgeführt wird, umfassend:
   die Aktivkohle wird der letzten Taurin-Mutterlauge unter temperatursenkenden Bedingungen zugesetzt, und die letzte Mutterlauge, die von Verunreinigungen befreit ist, wird nach der Feststoff-Flüssigkeits-Trennung erzeugt; die Lösung wird sauer oder neutral eingestellt.

5. Verfahren nach Anspruch 4, wobei der pH-Wert der Lösung in Abhängigkeit von der Laugensäure oder dem Kationenaustauscherharz eingestellt wird.

6. Verfahren nach einem der Ansprüche 1 bis 5, wobei die temperatursenkende Bedingung bedeutet, dass die Temperatur des Produktionssystems niedriger ist als die Temperatur des vorangegangenen Schritts, und die Verarbeitungstemperatur des Systems zwischen 15 und 25 °C geregelt wird.

7. Verfahren zur Herstellung von Ethylenoxid-Taurin, das die folgenden Schritte umfasst:

   S1: Ethylenoxid reagiert mit Natriumbisulfitlösung unter Bildung von Natriumisethionat;
   S2: Das aus S1 gewonnene Natriumisethionat und die nach Entfernung der Verunreinigungen zurückgewonnene Mutterlauge werden einer Ammonolyse-Reaktion unterzogen und durch Eindampfen konzentriert, um eine Natriumtaurinlösung zu erzeugen;
   S3: Die in S2 erzeugte Natriumtaurinlösung wird mit saurem Kationenaustauscherharz behandelt, um Taurinlauge zu erzeugen, die dann konzentriert und kristallisiert wird, um Rohtaurin und Mutterlauge zu erzeugen; oder Taurinkristallisationslauge wird durch Neutralisation mit Schwefelsäure erzeugt, Rohtaurin und Mutterlauge werden nach Abkühlung und Kristallisation erzeugt;
   S4: Die in S3 gesammelte Mutterlauge wird mehrfach konzentriert und kristallisiert, das Taurin abgetrennt und extrahiert und konzentriert, um die letzte Taurin-Mutterlauge zu erzeugen;
   wobei eines der Verfahren zur Entfernung von Verunreinigungen aus Taurin-Mutterlauge gemäß den Ansprüchen 1 bis 6 verwendet wird, um Verunreinigungen aus der letzten Mutterlauge in S4 zu entfernen, und die Mutterlauge nach der Entfernung von Verunreinigungen und der Rückgewinnung zum Ammonolyse-Reaktionsschritt von S2 geschickt wird.

8. System zur Entfernung von Verunreinigungen und Rückgewinnung von Taurin-Mutterlauge zur Behandlung der letzten Taurin-Mutterlauge im Ethylenoxid-Verfahren des Taurin-Herstellungsverfahrens, **dadurch gekennzeichnet, dass** es nacheinander eine Anionenharz-Adsorptionsvorrichtung zur Adsorption wirksamer Bestandteile, die Ammoniak-Misch- und Entsalzungsvorrichtung umfasst, die Zuführungsöffnung der Anionenharz-Adsorptionsvorrichtung mit dem Auslass der letzten Taurin-Mutterlauge, die im vorhergehenden Schritt erzeugt wurde, verbunden ist, die Anionenharz-Adsorptionsvorrichtung mit einer Anionenaustauscherharzsäule versehen ist, die Ammoniak-Misch- und Entsalzungsvorrichtung einen Ammoniak-Mischreaktionstank umfasst, der mit einem Zirkulationsweg und einer abgedichteten Filtervorrichtung versehen ist.

9. System zur Entfernung von Verunreinigungen nach Anspruch 8, wobei eine Vorrichtung zur Entfärbung und Entfernung von Verunreinigungen mit Aktivkohle zwischen der Anionenharz-Adsorptionsvorrichtung und dem Auslass der letzten Taurin-Mutterlauge vorgesehen ist, wobei die Vorrichtung zur Entfärbung und Entfernung von Verunreinigungen mit Aktivkohle einen Entfärbungstank und eine Filtervorrichtung umfasst.

10. System zur Entfernung von Verunreinigungen nach Anspruch 9, wobei eine Vorrichtung zur Adsorption von kationischem Harz mit dem vorderen oder hinteren Ende der Vorrichtung zur Entfärbung und Entfernung von Verunreinigungen durch Aktivkohle verbunden ist und die Vorrichtung zur Adsorption von kationischem Harz zur Senkung des pH-Werts der letzten Taurinmutterlauge verwendet wird.

11. System zur Entfernung von Verunreinigungen nach Anspruch 10, wobei, wenn die Adsorptionsvorrichtung für kationische Harze mit dem hinteren Ende der Vorrichtung zur Entfärbung von Aktivkohle und zur Entfernung von Verunreinigungen verbunden ist, die Adsorptionsvorrichtung für kationische Harze einen Rohstofftank und eine Kationenaustauscherharzsäule umfasst, der Rohstofftank mit der Auslassöffnung der Vorrichtung zur Entfärbung von Aktivkohle und zur Entfernung von Verunreinigungen verbunden ist und die Kationenaustauscherharzsäule mit der Anionenaustauscherharzsäule verbunden ist.

12. System zur Entfernung von Verunreinigungen nach einem der Ansprüche 8 bis 11, wobei die Anionenaustauscherharzsäule die Zugabe von alkalischer Lauge durch ein Regenerationszufuhrventil steuert und die Anionen auf der Anionenaustauscherharzsäule absorbiert und die absorbierten Anionen eluiert und in einem Mutterlaugenaufnahmetank durch eine Regenerationsweise gesammelt werden; der Auslass des Mutterlaugenaufnahmetanks ist mit dem Zufuhranschluss der Ammoniak-Misch- und Entsalzungsvorrichtung verbunden.

13. System zur Entfernung von Verunreinigungen nach Anspruch 12, wobei der Ammoniak-Mischreaktionstank mit einem Ammoniakeinlass, einem Zufuhreinlass und einem Auslass versehen ist und der Auslass des Ammoniak-Mischreaktionstanks über eine Pumpe mit der Zuführöffnung der abgedichteten Filtriervorrichtung verbunden ist und die Pumpe mit einem Transferauslassventil zum Auslassen des gefilterten klaren Materials versehen ist.

**Revendications**

1. Procédé d'élimination des impuretés de la liqueur mère de taurine et de récupération de la liqueur mère de taurine, qui est utilisée pour le processus de production de taurine dans le processus d'oxyde d'éthylène pour traiter la dernière liqueur mère de taurine, comprenant les étapes suivantes :

   (a) la dernière liqueur mère de taurine est échangée par ion à travers une résine échangeuse d'anions, et les composants efficaces sont adsorbés sur la résine échangeuse d'anions ; la résine échangeuse d'anions est ensuite éluée et régénérée avec une solution alcaline, et la liqueur de sortie est collectée ;
   (b) un traitement de mélange à l'ammoniac est effectué sur la liqueur de sortie collectée à l'étape (a), et une séparation solide-liquide est effectuée pour générer une liqueur mère après élimination des impuretés ;
   (c) la liqueur mère générée à l'étape b) entre dans l'étape d'ammonolyse.

2. Procédé selon la revendication 1, dans lequel pendant ledit traitement de mélange d'ammoniac, de l'ammoniac de liqueur ou du gaz d'ammoniac est ajouté à ladite liqueur de matériau de sortie, et le rapport masse-volume de l'ammoniac est de 15g/100ml ou plus.

3. Procédé selon la revendication 2, dans lequel ladite résine échangeuse d'anions est une résine échangeuse d'anions alcalins.

4. Procédé selon la revendication 3, dans lequel avant l'étape (a), réalisation d'un traitement de décoloration et d'élimination des impuretés sur la dernière liqueur mère de taurine avec du charbon actif, comprenant :
le charbon actif est ajouté à la dernière liqueur mère de taurine dans des conditions de réduction de la température, et la dernière liqueur mère débarrassée des impuretés est générée après la séparation solide-liquide ; ajuster la solution en acide ou en neutre.

5. Procédé selon la revendication 4, dans lequel le pH de la solution est ajusté en fonction de l'acide de la liqueur ou de la résine échangeuse de cations.

6. Procédé selon l'une quelconque des revendications 1 à 5, dans lequel la condition de réduction de la température signifie que la température du système de production est inférieure à la température de l'étape précédente, et que la température de traitement du système est commandée entre 15 et 25°C.

7. Procédé de production de taurine à partir d'oxyde d'éthylène, comprenant les étapes suivantes :

S1 : l'oxyde d'éthylène réagit avec une solution de bisulfite de sodium pour générer de l'iséthionate de sodium ;
S2 : l'iséthionate de sodium généré à partir de S1 et la liqueur mère récupérée après élimination des impuretés sont soumis à une réaction d'ammonolyse et concentrés par évaporation pour générer une solution de taurine de sodium ;
S3 : la solution de taurine de sodium générée en S2 est traitée avec une résine échangeuse de cations acide pour générer une liqueur de taurine, qui est ensuite concentrée et cristallisée pour générer la taurine brute et la liqueur mère ; ou la liqueur de cristallisation de la taurine est générée par neutralisation avec de l'acide sulfurique, la taurine brute et la liqueur mère sont générées après refroidissement et cristallisation ;
S4 : concentrer et cristalliser la liqueur mère recueillie en S3 plusieurs fois, séparer et extraire la taurine, et concentrer pour générer la dernière liqueur mère de taurine ;
dans lequel, l'un quelconque des procédés d'élimination des impuretés de la liqueur mère de taurine selon les revendications 1 à 6 est utilisé pour éliminer les impuretés de la dernière liqueur mère en S4, et la liqueur mère après élimination des impuretés et récupération est envoyée à l'étape de réaction d'ammonolyse de S2.

8. Système d'élimination des impuretés et de récupération de la liqueur mère de taurine pour traiter la dernière liqueur mère de taurine dans le processus d'oxyde d'éthylène du processus de production de taurine, dans lequel il comprend consécutivement un dispositif d'adsorption de résine anionique pour adsorber les ingrédients efficaces, le dispositif de mélange et de désalinisation de l'ammoniac, l'orifice d'alimentation dudit dispositif d'adsorption à résine anionique est relié à la sortie de la dernière liqueur mère de taurine générée à l'étape précédente, ledit dispositif d'adsorption à résine anionique est pourvu d'une colonne de résine échangeuse d'anions, ledit dispositif de mélange et de désalinisation de l'ammoniac comprend un réservoir de réaction de mélange de l'ammoniac pourvu d'une voie de circulation et d'un dispositif de filtrage scellé.

9. Système d'élimination des impuretés selon la revendication 8, dans lequel un dispositif de décoloration et d'élimination des impuretés sur charbon actif est prévu entre ledit dispositif d'adsorption sur résine anionique et la sortie de la dernière liqueur mère de taurine, ledit dispositif de décoloration et d'élimination des impuretés sur charbon actif comprend un réservoir de décoloration et un dispositif de filtrage.

10. Système d'élimination des impuretés selon la revendication 9, dans lequel un dispositif d'adsorption à résine cationique est connecté à l'extrémité avant ou à l'extrémité arrière dudit dispositif de décoloration au charbon actif et d'élimination des impuretés et ledit dispositif d'adsorption à résine cationique est utilisé pour réduire la valeur du pH de la dernière liqueur mère de taurine.

11. Système d'élimination des impuretés selon la revendication 10, dans lequel lorsque le triste dispositif d'adsorption de résine cationique est connecté à l'extrémité arrière dudit dispositif de décoloration au charbon actif et d'élimination des impuretés, ledit dispositif d'adsorption de résine cationique comprend un réservoir de matières premières et une colonne de résine échangeuse de cations, ledit réservoir de matières premières est connecté à l'orifice de décharge dudit dispositif de décoloration au charbon actif et d'élimination des impuretés, et ladite colonne de résine échangeuse de cations est connectée à ladite colonne de résine échangeuse d'anions.

**12.** Système d'élimination des impuretés selon l'une des revendications 8 à 11, dans lequel ladite colonne de résine échangeuse d'anions commande l'ajout de liqueur alcaline par l'intermédiaire d'une vanne d'alimentation de régénération et absorbe les anions sur ladite colonne de résine échangeuse d'anions, et les anions absorbés sont élués et recueillis dans un réservoir de réception de liqueur mère par une méthode de régénération ; la sortie dudit réservoir de réception de liqueur mère est reliée à l'orifice d'alimentation dudit dispositif de mélange et de désalinisation de l'ammoniaque.

**13.** Système d'élimination des impuretés selon la revendication 12, dans lequel ledit réservoir de réaction de mélange d'ammoniac est pourvu d'une entrée d'ammoniac, d'une entrée d'alimentation et d'une sortie de décharge, et la sortie de décharge dudit réservoir de réaction de mélange d'ammoniac est reliée à l'orifice d'alimentation dudit dispositif de filtrage étanche par l'intermédiaire d'une pompe, et ladite pompe est pourvue d'une vanne de décharge de transfert pour décharger la matière claire filtrée.

Fig. 1

Fig. 2

Fig. 3

Fig. 4

Fig. 5

Figure 6

Figure 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101508657
- CN 10158658
- CN 10158659
- CN 101486669
- CN 107056659 A
- EP 3415497 A1
- CN 105732440
- CN 107056659

**Non-patent literature cited in the description**

- **LIU FUMING ; XIE LIMIN.** Research on Taurine Ammonolysis Process. *Shandong Chemical Industry,* 2015, vol. 44